# EUROPEAN PATENT APPLICATION

(11) **EP 0 906 953 A1**
(43) Date of publication of application: **07.04.1999**
(21) Application number: 97116061.9
(22) Date of filing: 16.09.1997
(51) Int. Cl.: C12N 15/12, C07K 14/47, A61K 38/17, C12N 5/20

(54) **Allelic variant of human stat3**

(71) Applicant: APPLIED RESEARCH SYSTEMS ARS HOLDING N.V., Curaçao (AN)
(72) Inventor: Serlupi-Crescenzi, Ottaviano, 00186 Rome (IT); Della Pietra, Linda, 00162 Rome (IT)
(74) Representative: Vannini, Mario

(57) **Abstract**

A predominant allelic variant of the human STAT3 protein, the cDNA sequence encoding it, its use in therapy and/or in diagnosis of autoimmune and/or inflammatory diseases, as well as pharmaceutical compositions comprising it.

SEQUENCE LISTING

(1) GENERAL INFORMATION:

(i) APPLICANT:
(A) NAME: APPLIED RESEARCH SYSTEMS ARS HOLDING N.V.
(B) STREET: 14, JOHN B. GORSIRAWEG
(C) CITY: CURACAO
(E) COUNTRY: THE NETHERLANDS ANTILLES
(F) POSTAL CODE (ZIP): NONE
(G) TELEPHONE: 599-9-639300
(H) TELEFAX: 599-9-614129

(ii) TITLE OF INVENTION: HUMAN STAT3 ALLELE

(iii) NUMBER OF SEQUENCES: 13

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 423 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(v) FRAGMENT TYPE: internal

(ix) FEATURE:
(A) NAME/KEY: CDS
(B) LOCATION:1..423

(xi) SEQUENCE DESCRIPTION: SEQ ID NO: 1:

TGG CTA GAC AAT ATC ATC GAC CTT GTG AAA AAG TAT ATC TTG GCC CTT 48
Trp Leu Asp Asn Ile Ile Asp Leu Val Lys Lys Tyr Ile Leu Ala Leu
1               5                   10                  15

TGG AAT GAA GGG TAC ATC ATG GGT TTC ATC AGC AAG GAG CGG GAG CGG          96
Trp Asn Glu Gly Tyr Ile Met Gly Phe Ile Ser Lys Glu Arg Glu Arg
                20                  25                  30

GCC ATC TTG AGC ACT AAG CCC CCA GGC ACC TTC CTG CTG CGC TTC AGT         144
Ala Ile Leu Ser Thr Lys Pro Pro Gly Thr Phe Leu Leu Arg Phe Ser
            35                  40                  45

GAA AGC AGC AAA GAA GGA GGC GTC ACT TTC ACT TGG GTG GAG AAG GAC         192
Glu Ser Ser Lys Glu Gly Gly Val Thr Phe Thr Trp Val Glu Lys Asp
        50                  55                  60

ATC AGC GGT AAG ACC CAG ATC CAG TCC GTG GAA CCA TAC ACA AAG CAG         240
Ile Ser Gly Lys Thr Gln Ile Gln Ser Val Glu Pro Tyr Thr Lys Gln
 65                  70                  75                  80

CAG CTG AAC AAC ATG TCA TTT GCT GAA ATC ATC ATG GGC TAT AAG ATC         288
Gln Leu Asn Asn Met Ser Phe Ala Glu Ile Ile Met Gly Tyr Lys Ile
                 85                  90                  95

ATG GAT GCT ACC AAT ATC CTG TTG TCT CCA CTT GTC TAT CTC TAT CCT         336
Met Asp Ala Thr Asn Ile Leu Leu Ser Pro Leu Val Tyr Leu Tyr Pro
            100                 105                 110

GAC ATT CCC AAG GAG GAG GCA TTC GGG AAG TAT TGT CGG CCA GAG AGC         384
Asp Ile Pro Lys Glu Glu Ala Phe Gly Lys Tyr Cys Arg Pro Glu Ser
        115                 120                 125

CAG GAG CAT CCT GAA GCT GAC CCA GGT AGC GCT GCC CCA                     423
Gln Glu His Pro Glu Ala Asp Pro Gly Ser Ala Ala Pro
    130                 135                 140


(2) INFORMATION FOR SEQ ID NO: 2:

     (i) SEQUENCE CHARACTERISTICS:
          (A) LENGTH: 141 amino acids
          (B) TYPE: amino acid
          (D) TOPOLOGY: linear

    (ii) MOLECULE TYPE: protein
    (xi) SEQUENCE DESCRIPTION: SEQ ID NO: 2:

Trp Leu Asp Asn Ile Ile Asp Leu Val Lys Lys Tyr Ile Leu Ala Leu
  1               5                  10                  15

Trp Asn Glu Gly Tyr Ile Met Gly Phe Ile Ser Lys Glu Arg Glu Arg
                20                  25                  30

Ala Ile Leu Ser Thr Lys Pro Pro Gly Thr Phe Leu Leu Arg Phe Ser
            35                  40                  45

Glu Ser Ser Lys Glu Gly Gly Val Thr Phe Thr Trp Val Glu Lys Asp
    50                  55                  60

Ile Ser Gly Lys Thr Gln Ile Gln Ser Val Glu Pro Tyr Thr Lys Gln
65                  70                  75                  80

Gln Leu Asn Asn Met Ser Phe Ala Glu Ile Ile Met Gly Tyr Lys Ile
                85                  90                  95

Met Asp Ala Thr Asn Ile Leu Leu Ser Pro Leu Val Tyr Leu Tyr Pro
            100                 105                 110

Asp Ile Pro Lys Glu Glu Ala Phe Gly Lys Tyr Cys Arg Pro Glu Ser
        115                 120                 125

Gln Glu His Pro Glu Ala Asp Pro Gly Ser Ala Ala Pro
    130                 135                 140

(2) INFORMATION FOR SEQ ID NO: 3:

     (i) SEQUENCE CHARACTERISTICS:
          (A) LENGTH: 423 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

    (iv) ANTI-SENSE: NO

     (v) FRAGMENT TYPE: internal

    (ix) FEATURE:
          (A) NAME/KEY: CDS
          (B) LOCATION:1..423

    (xi) SEQUENCE DESCRIPTION: SEQ ID NO: 3:

TGG CTA GAC AAT ATC ATC GAC CTT GTG AAA AAG TAC ATC CTG GCC CTT      48
Trp Leu Asp Asn Ile Ile Asp Leu Val Lys Lys Tyr Ile Leu Ala Leu
  1               5                  10                  15

TGG AAC GAA GGG TAC ATC ATG GGC TTT ATC AGT AAG GAG CGG GAG CGG      96
Trp Asn Glu Gly Tyr Ile Met Gly Phe Ile Ser Lys Glu Arg Glu Arg
             20                  25                  30

GCC ATC TTG AGC ACT AAG CCT CCA GGC ACC TTC CTG CTA AGA TTC AGT     144
Ala Ile Leu Ser Thr Lys Pro Pro Gly Thr Phe Leu Leu Arg Phe Ser

        35                  40                  45

GAA AGC AGC AAA GAA GGA GGC GTC ACT TTC ACT TGG GTG GAG AAG GAC 192
Glu Ser Ser Lys Glu Gly Gly Val Thr Phe Thr Trp Val Glu Lys Asp
    50                  55                  60

ATC AGC GGT AAG ACC CAG ATC CAG TCC GTG GAA CCA TAC ACA AAG CAG 240
Ile Ser Gly Lys Thr Gln Ile Gln Ser Val Glu Pro Tyr Thr Lys Gln
65                  70                  75                  80

CAG CTG AAC AAC ATG TCA TTT GCT GAA ATC ATC ATG GGC TAT AAG ATC 288
Gln Leu Asn Asn Met Ser Phe Ala Glu Ile Ile Met Gly Tyr Lys Ile
                85                  90                  95

ATG GAT GCT ACC AAT ATC CTG GTG TCT CCA CTG GTC TAT CTC TAT CCT 336
Met Asp Ala Thr Asn Ile Leu Val Ser Pro Leu Val Tyr Leu Tyr Pro
            100                 105                 110

GAC ATT CCC AAG GAG GAG GCA TTC GGA AAG TAT TGT CGG CCA GAG AGC 384
Asp Ile Pro Lys Glu Glu Ala Phe Gly Lys Tyr Cys Arg Pro Glu Ser
        115                 120                 125

CAG GAG CAT CCT GAA GCT GAC CCA GGT AGC GCT GCC CCA 423
Gln Glu His Pro Glu Ala Asp Pro Gly Ser Ala Ala Pro
    130                 135                 140

(2) INFORMATION FOR SEQ ID NO: 4:

(i) SEQUENCE CHARACTERISTICS:
(A) LENGTH: 141 amino acids
(B) TYPE: amino acid
(D) TOPOLOGY: linear

(ii) MOLECULE TYPE: protein
(xi) SEQUENCE DESCRIPTION: SEQ ID NO: 4:

Trp Leu Asp Asn Ile Ile Asp Leu Val Lys Lys Tyr Ile Leu Ala Leu
1               5                   10                  15

Trp Asn Glu Gly Tyr Ile Met Gly Phe Ile Ser Lys Glu Arg Glu Arg
            20                  25                  30

Ala Ile Leu Ser Thr Lys Pro Pro Gly Thr Phe Leu Leu Arg Phe Ser
        35                  40                  45

Glu Ser Ser Lys Glu Gly Gly Val Thr Phe Thr Trp Val Glu Lys Asp
    50                  55                  60

Ile Ser Gly Lys Thr Gln Ile Gln Ser Val Glu Pro Tyr Thr Lys Gln
65                  70                  75                  80

Gln Leu Asn Asn Met Ser Phe Ala Glu Ile Ile Met Gly Tyr Lys Ile
                85                  90                  95

Met Asp Ala Thr Asn Ile Leu Val Ser Pro Leu Val Tyr Leu Tyr Pro
            100                 105                 110

Asp Ile Pro Lys Glu Glu Ala Phe Gly Lys Tyr Cys Arg Pro Glu Ser
        115                 120                 125

Gln Glu His Pro Glu Ala Asp Pro Gly Ser Ala Ala Pro
    130                 135                 140

(2) INFORMATION FOR SEQ ID NO: 5:

     (i) SEQUENCE CHARACTERISTICS:
          (A) LENGTH: 423 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

    (iv) ANTI-SENSE: NO

     (v) FRAGMENT TYPE: internal

    (ix) FEATURE:
          (A) NAME/KEY: CDS
          (B) LOCATION:1..423

    (xi) SEQUENCE DESCRIPTION: SEQ ID NO: 5:

TGG CTA GAC AAT ATC ATC GAC CTT GTG AAA AAG TAT ATC TTG GCC CTT          48
Trp Leu Asp Asn Ile Ile Asp Leu Val Lys Lys Tyr Ile Leu Ala Leu
  1               5                  10                  15

TGG AAT GAA GGG TAC ATC ATG GGT TTC ATC AGC AAG GAG CGG GAG CGG          96
Trp Asn Glu Gly Tyr Ile Met Gly Phe Ile Ser Lys Glu Arg Glu Arg
             20                  25                  30

GCC ATC CTA AGC ACA AAG CCC CCG GGC ACC TTC CTA CTG CGC TTC AGC         144
Ala Ile Leu Ser Thr Lys Pro Pro Gly Thr Phe Leu Leu Arg Phe Ser
         35                  40                  45

GAG AGC AGC AAA GAA GGA GGG GTC ACT TTC ACT TGG GTG GAA AAG GAC         192
Glu Ser Ser Lys Glu Gly Gly Val Thr Phe Thr Trp Val Glu Lys Asp
     50                  55                  60

ATC AGT GGC AAG ACC CAG ATC CAG TCT GTA GAG CCA TAC ACC AAG CAG 240
Ile Ser Gly Lys Thr Gln Ile Gln Ser Val Glu Pro Tyr Thr Lys Gln
65                  70                  75                  80

CAG CTG AAC AAC ATG TCA TTT GCT GAA ATC ATC ATG GGC TAT AAG ATC 288
Gln Leu Asn Asn Met Ser Phe Ala Glu Ile Ile Met Gly Tyr Lys Ile
                85                  90                  95

ATG GAT GCG ACC AAC ATC CTG GTG TCT CCA CTT GTC TAC CTC TAC CCC 336
Met Asp Ala Thr Asn Ile Leu Val Ser Pro Leu Val Tyr Leu Tyr Pro
            100                 105                 110

GAC ATT CCC AAG GAG GAG GCA TTT GGA AAG TAC TGT AGG CCC GAG AGC 384
Asp Ile Pro Lys Glu Glu Ala Phe Gly Lys Tyr Cys Arg Pro Glu Ser
        115                 120                 125

CAG GAG CAC CCC GAA GCC GAC CCA GGT AGC TCT GCC CCA 423
Gln Glu His Pro Glu Ala Asp Pro Gly Ser Ser Ala Pro
    130                 135                 140

(2) INFORMATION FOR SEQ ID NO: 6:

(i) SEQUENCE CHARACTERISTICS:
(A) LENGTH: 141 amino acids
(B) TYPE: amino acid
(D) TOPOLOGY: linear

(ii) MOLECULE TYPE: protein
(xi) SEQUENCE DESCRIPTION: SEQ ID NO: 6:

Trp Leu Asp Asn Ile Ile Asp Leu Val Lys Lys Tyr Ile Leu Ala Leu
1               5                   10                  15

Trp Asn Glu Gly Tyr Ile Met Gly Phe Ile Ser Lys Glu Arg Glu Arg
            20                  25                  30

Ala Ile Leu Ser Thr Lys Pro Pro Gly Thr Phe Leu Leu Arg Phe Ser
        35                  40                  45

Glu Ser Ser Lys Glu Gly Gly Val Thr Phe Thr Trp Val Glu Lys Asp
    50                  55                  60

Ile Ser Gly Lys Thr Gln Ile Gln Ser Val Glu Pro Tyr Thr Lys Gln
65                  70                  75                  80

Gln Leu Asn Asn Met Ser Phe Ala Glu Ile Ile Met Gly Tyr Lys Ile
                85                  90                  95

Met Asp Ala Thr Asn Ile Leu Val Ser Pro Leu Val Tyr Leu Tyr Pro
            100                 105                 110

Asp Ile Pro Lys Glu Glu Ala Phe Gly Lys Tyr Cys Arg Pro Glu Ser
        115                 120                 125

Gln Glu His Pro Glu Ala Asp Pro Gly Ser Ser Ala Pro
    130                 135                 140

(2) INFORMATION FOR SEQ ID NO: 7:

     (i) SEQUENCE CHARACTERISTICS:
          (A) LENGTH: 22 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

    (iv) ANTI-SENSE: NO

    (xi) SEQUENCE DESCRIPTION: SEQ ID NO: 7:

TGAAGGGTAC ATCATGGGTT TC                                                22

(2) INFORMATION FOR SEQ ID NO: 8:

     (i) SEQUENCE CHARACTERISTICS:
          (A) LENGTH: 29 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

    (iv) ANTI-SENSE: NO

    (xi) SEQUENCE DESCRIPTION: SEQ ID NO: 8:

TCAGGATAGA GATAGACAAG TGGAGACAA                                         29

(2) INFORMATION FOR SEQ ID NO: 9:

     (i) SEQUENCE CHARACTERISTICS:
          (A) LENGTH: 27 base pairs
          (B) TYPE: nucleic acid
          (C) STRANDEDNESS: single
          (D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO: 9:

CCTCCTTCTT TGCTGCTTTC ACTGAAG 27

(2) INFORMATION FOR SEQ ID NO: 10:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO: 10:

CGAAGGGTAC ATCATGGGCT TT 22

(2) INFORMATION FOR SEQ ID NO: 11:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO: 11:

CCTCCTTCTT TGCTGCTTTC ACTGAATCTT 30

(2) INFORMATION FOR SEQ ID NO: 12:

(i) SEQUENCE CHARACTERISTICS:
(A) LENGTH: 33 base pairs
(B) TYPE: nucleic acid

(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO: 12:

TGAAGGGTAC ATCATGGGTT TCATCAGTAA GGA 33

(2) INFORMATION FOR SEQ ID NO: 13:

(i) SEQUENCE CHARACTERISTICS:
(A) LENGTH: 37 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(xi) SEQUENCE DESCRIPTION: SEQ ID NO: 13:

TCAGGATAGA GATAGACAAG TGGAGACAAC AGGATAT 37

## Description

### FIELD OF THE INVENTION

The present invention relates to a human STAT3 allelic variant, the cDNA sequence encoding it, its use in therapy and/or in diagnosis of autoimmune and/or inflammatory diseases, as well as pharmaceutical compositions comprising it.

### BACKGROUND OF THE INVENTION

Signal Transducer and Activator of Transcription (STAT) proteins are a new class of intracellular transcription factors which play an essential function in the cellular responses to cytokines (Stahl et al., 1994; Gouilleux et al., 1995; Azam et al., 1995; Tian et al., 1994; May et al., 1996; and Iwatsuki et al., 1997).

Most of these proteins have been well characterized by sequencing, and their structure as well as the mechanism of their actions has been extensively analyzed and well documented (Wegenka et al., 1993; Akira et al., 1994; Wegenka et al., 1994; Quelle et al., 1995 ad Silva et al., 1996).

These proteins contain SH2 and SH3 domains as well as a phosphorylation site at their carboxy-terminal region (Kapetein et al., 1996; and Herman et al., 1996). After cytokine receptor activation through ligand binding, the intracellular portion of the receptor becomes phosphorylated by an associated kinase of the JAK family.

STAT proteins then bind to the phosphorylated receptor, through their SH2 domain, and are in turn phosphorylated by JAKs (Stahl et al., 1995). Phosphorylated STAT proteins then dimerize and translocate to the nucleus, where they are able to recognize specific DNA responsive elements (Seidel et al., 1995; and Harroch et al., 1994).

STAT3 has been identified as a important mediator of the signal imparted by the IL-6 family of cytokines, as well as by EGF and by a number of other interleukins and growth factors.

STAT3 has been shown to play a central role in the upregulation of hepatic acute-phase proteins (Wegenka et al., 1993; and Zhang et al., 1996) in the growth arrest of monocytic cells (Yamanaka et al., 1996; and Minami et al., 1996) as well as in the survival of myeloma cells (Harroch et al., 1994).

The critical role played by STAT3 in cellular function is emphasized by the very high sequence conservation in mammalian species. Human STAT3 shares 98-99% similarity at the amino acid level with its murine or rat counterpart.

### DESCRIPTION OF THE INVENTION

During experiments on the analysis of STAT3 interactions, we have amplified by RT-PCR from HepG2 cells a cDNA fragment corresponding to the SH2 domain of human STAT3. We have found by DNA sequencing that the SH2 domain we have isolated shows a divergence of 13 residues over the corresponding sequence of the original published human STAT3 gene (Akira et al., 1994).

In order to determine the nature of this sequence variant, we have designed three pairs of primers with 3' ends corresponding to nucleotide positions at variance between the two human cDNA sequences.

Upon such investigations it resulted that the new variant corresponds to at least the most frequent allele of human STAT3.

Therefore, the main object of this invention is the above-mentioned allelic variant of human STAT3 protein. In particular, the object of the invention is a polypeptide comprising the amino acid sequence of SEQ ID NO: 4, or a functionally equivalent salt, or a functionally equivalent derivative, or an active fraction, or a fusion protein.

The definition "salt" as used herein refers both to salts of the carboxyl-groups and to the salts of the amino functions of the compound obtainable through known methods. The salts of the carboxyl-groups comprise inorganic salts as, for example, sodium, potassium, calcium salts and salts with organic bases as those formed with a amine as triethanolamine, arginine or lisine. The salts of the amino groups comprise for example salts with inorganic acids as hydrochloric acid and with organic acids as acetic acid.

The definition "derivative" as herein used refers to derivatives which can be prepared from the functional groups present on the lateral chains of the amino acid moieties or on the terminal N- or C- groups according to known methods and are comprised in the invention when they are pharmaceutically acceptable i.e. when they do not destroy the protein activity or do not impart toxicity to the pharmaceutical compositions containing them. Such derivatives include for example esters or aliphatic amides of the carboxyl-groups and N-acyl derivatives of free amino groups or O-acyl derivatives of free hydroxyl-groups and are formed with acyl-groups as for example alcanoyl- or aroyl-groups.

As "active fraction" of the protein the present invention refers to any fragment or precursor of the polypeptidic chain of the compound itself, alone or in combination with related molecules or residues bound to it, for example residues of sugars or phosphates, or aggregates of the polypeptide molecule when such fragments or precursors show the same activity of the protein of the invention, as medicament.

The definition "fusion protein" as herein used refers to polypeptides comprising the polypeptide of the invention above specified fused with another protein and having a longer lasting half-life in body fluids. It can for example be fused with another protein such as, for example, an immunoglobulin.

Another object of the invention is the DNA molecule comprising the DNA sequence coding for the allelic variant of the invention, including nucleotide sequences substantially the same.

"Nucleotide sequences substantially the same" includes all other nucleic acid sequences which, by virtue of the degeneracy of the genetic code, also code for the given amino acid sequences. In particular, the present invention refers to the nucleotide sequence comprising the SEQ ID NO: 3.

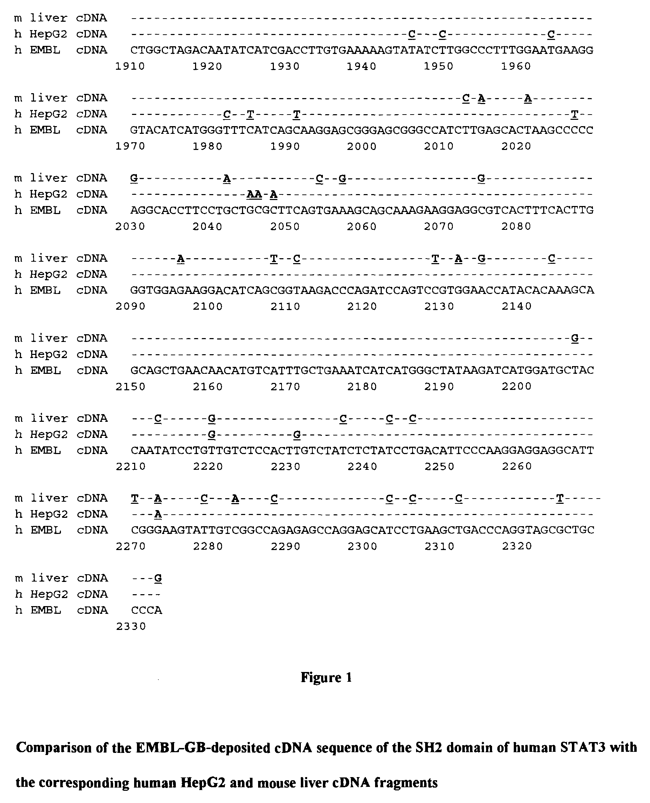

The present invention also refers to recombinant DNA molecules which hybridize with the DNA sequence coding for the above-mentioned allelic variant of hSTAT3 and whose nucleotide sequences show at least the same 13 differences in the SH2 domain (with respect to the human STAT3 sequence in Akira et al., 1994), as shown in Figure 1. The gene can contain, or not, the natural introns and can be obtained for example by extraction from appropriate cells and purification with known methods.

Furthermore, the present invention also includes recombinant DNA molecules which hybridize under stringent conditions with a probe having a nucleotide sequence selected between SEQ ID NO: 10 and SEQ ID NO: 11.

The invention also includes expression vectors which comprise the above DNAs, host-cells transformed with such vectors and a process of preparation of such allelic variant of hSTAT3, its active fragments or fusion proteins, through the culture in appropriate culture media of said transformed cells.

The DNA sequence coding for the protein of the invention can be inserted and ligated into a suitable plasmid. Once formed, the expression vector is introduced into a suitable host cell, which then expresses the vector(s) to yield the desired protein.

Expression of any of the recombinant proteins of the invention as mentioned herein can be effected in eukaryotic cells (e.g. yeasts, insect or mammalian cells) or prokaryotic cells, using the appropriate expression vectors. Any method known in the art can be employed.

For example the DNA molecules coding for the proteins obtained by any of the above methods are inserted into appropriately constructed expression vectors by techniques well known in the art (see Sambrook et al, 1989). Double stranded cDNA is linked to plasmid vectors by homopolymeric tailing or by restriction linking involving the use of synthetic DNA linkers or blunt-ended ligation techniques: DNA ligases are used to ligate the DNA molecules ad undesirable joining is avoided by treatment with alkaline phosphatase.

In order to be capable of expressing the desired protein, an expression vector should comprise also specific nucleotide sequences containing transcriptional and translational regulatory information linked to the DNA coding the desired protein in such a way as to permit gene expression and production of the protein. First in order for the gene to be transcribed, it must be preceded by a promoter recognizable by RNA polymerase, to which the polymerase binds and thus initiates the transcription process. There are a variety of such promoters in use, which work with different efficiencies (strong and weak promoters).

For eukaryotic hosts, different transcriptional and translational regulatory sequences may be employed, depending on the nature of the host. They may be derived form viral sources, such as adenovirus, bovine papilloma virus, Simian virus or the like, where the regulatory signals are associated with a particular gene which has a high level of expression. Examples are the TX promoter of the Herpes virus, the SV40 early promoter, the yeast gal4 gene promoter, etc. Transcriptional initiation regulatory signals may be selected which allow for repression and activation, so that expression of the genes can be modulated.

The DNA molecule comprising the nucleotide sequence coding for the protein of the invention is inserted into vector(s), having the operably linked transcriptional and translational regulatory signals, which is capable of integrating the desired gene sequences into the host cell.

The cells which have been stably transformed by the introduced DNA can be selected by also introducing one or more markers which allow for selection of host cells which contain the expression vector. The marker may also provide for phototrophy to a auxotropic host, biocide resistance, e.g. antibiotics, or heavy metals such as copper, or the like. The selectable marker gene can either be directly linked to the DNA gene sequences to be expressed, or introduced into the same cell by co-transfection. Additional elements may also be needed for optimal synthesis of proteins of the invention.

Factors of importance in selecting a particular plasmid or viral vector include: the ease with which recipient cells, that contain the vector may be recognized and selected form those recipient cells which do not contain the vector; the number of copies of the vector which are desired in a particular host; and whether it is desirable to be able to "shuttle" the vector between host cells of different species.

Once the vector(s) or DNA sequence containing the construct(s) has been prepared for expression the DNA construct(s) mat be introduced into an appropriate host cell by any of a variety of suitable means: transformation, transfection, conjugation, protoplast fusion, electroporation, calcium phosphate-precipitation, direct microinjection, etc.

Host cells may be either prokaryotic or eukaryotic. Preferred are eukaryotic hosts, e.g. mammalian cells, such as human, monkey, mouse, and Chinese hamster ovary (CHO) cells, because they provide post-translational modifications to protein molecules, including correct folding or glycosylation at correct sites. Also yeast cells can carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products ad secretes peptides bearing leader sequences (i.e., pre-peptides).

After the introduction of the vector(s), the host cells are grown in a selective medium, which selects for the growth of vector-containing cells. Expression of the cloned gene sequence(s) results in the production of the desired proteins.

Purification of the recombinant proteins is carried out by any one of the methods known for this purpose, i.e. any conventional procedure involving extraction, precipitation, chromatography, electrophoresis, or the like. A further purification procedure that may be used in preference for purifying the protein of the invention is affinity chromatography using monoclonal antibodies which bind the target protein and which are produced and immobilized on a gel matrix: contained within a column. Impure preparations containing the recombinant protein are passed through the column. The protein will be bound to the column by the specific antibody while the impurities will pass through. After washing, the protein is eluted from the gel by a change in pH or ionic strength.

As already stated, the protein of the invention is useful in the therapy and/or diagnosis of autoimmune and/or inflammatory diseases. Therefore, in a further aspect, the present invention provides the use of the protein of the invention in the manufacture of a medicament for the treatment of autoimmune diseases and/or inflammatory diseases.

The medicament is preferably presented in the form of a pharmaceutical composition comprising the protein of the invention together with one or more pharmaceutically acceptable carriers and/or excipients. Such pharmaceutical compositions form yet a further aspect of the present invention.

The invention will now be described by means of the following Examples, which should not be construed as in any way limiting the present invention. The Examples will refer to the Figures specified here below.

### DESCRIPTION OF THE FIGURES

Figure 1: It shows a comparison of the EMBL-GB-deposited cDNA sequence of the SH2 domain of human STAT3 with the corresponding human HepG2 and mouse liver cDNA fragments. The full 424 bp nucleotide sequence and its numbering are taken from the human STAT3 cDNA sequence deposited in the EMBL-GB databases, while nucleotides of human HepG2 (from this patent application) and mouse liver cDNAs, which differ from the deposited human cDNA sequence, are reported above the full sequence, in bold and underlined.

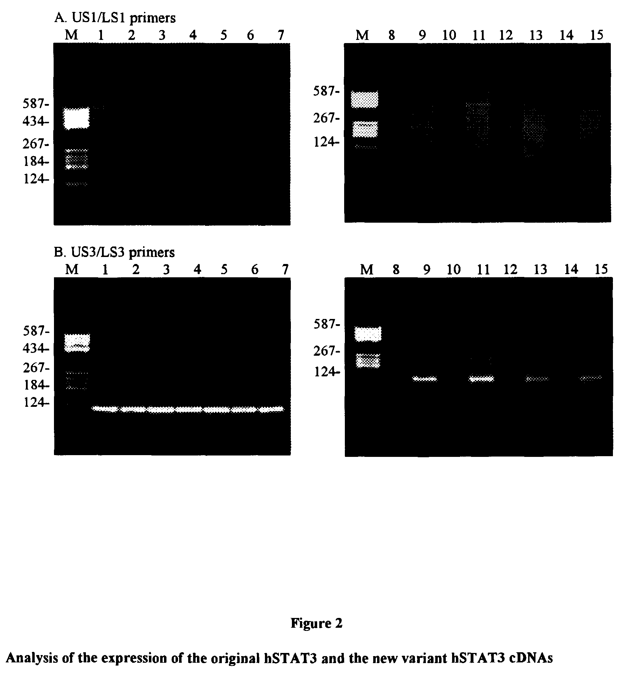

Figure 2. It represents an analysis of the expression of the original hSTAT3 and the new variant hSTAT3 cDNAs. RNA was extracted with the Trizol reagent, reverse-transcribed with oligo (dT) and the analytical PCR reaction was carried out with the Taq polymerase in capillary tubes, as described in the Materials and methods section.
   **A.** PCR products amplified with the US1/LS1 pair of primers specific for the original published hSTAT3 sequence.
   **B.** PCR products amplified with the US3/LS3 pair of primers specific for the new hSTAT3 sequence variant found in this study. Lanes: M) Molecular size markers. 1) Liver RNA. 2) Spleen RNA. 3) Uterus RNA. 4) Lung RNA. 5) Skin RNA. 6) RNA from cord blood cells. 7) Dermal fibroblasts RNA. 8) Heart RNA with no reverse transcriptase. 9) Heart RNA. 10) Fetal liver RNA with no reverse transcriptase. 11) Fetal liver RNA. 12) Small intestine RNA with no reverse transcriptase. 13) Small intestine RNA. 14) Placental RNA with no reverse transcriptase. 15) Placental RNA.

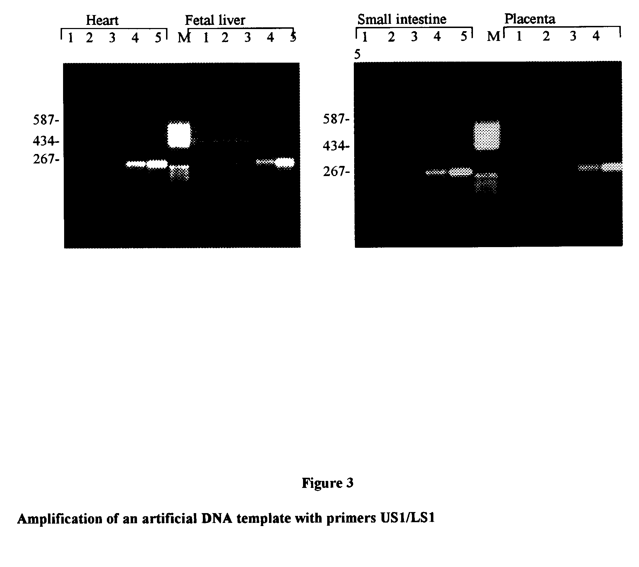

Figure 3. It shows the amplification of an artificial DNA template with primers US1/LS1. The artificial DNA template composed of the hSTAT3 variant sequence flagment flanked at its 5' end by the US1 primer sequence and at its 3' end by the LS1 primer sequence, was created by preparative PCR from HepG2 cDNA, using primers US4/LS4, as described in the Materials and Methods section. The artificial template was fractionated in 2% agarose gel and the relevant band of 285 bp was purified with the agarose gel DNA extraction kit (Boeringer Mannheim, Mannheim, Germany). This template was then spiked at various concentrations to 1 µl of the relevant cDNA (originated from approximately 100 ng of the corresponding RNA). Lanes: M) Molecular size markers. 1) No spiking. 2) 0.3 fg of artificial template spiked. 3) 3 fg of artificial template spiked. 4) 30 fg of artificial template spiked. 5) 300 fg of artificial template spiked.

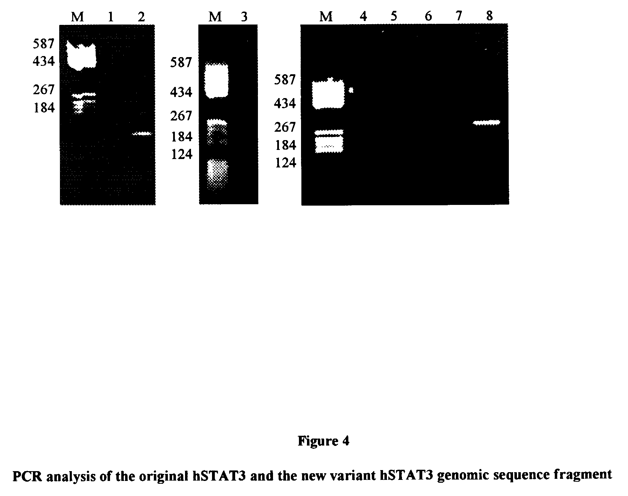

Figure 4. It represents the PCR analysis of the original hSTAT3 and the new variant hSTAT3 genomic sequence flagment. 40 ng of human genomic DNA were used in analytical PCR reactions carried out in capillary tubes, as described in the Materials and methods section. Lanes: M) Molecular size markers. 1) Genomic DNA amplified with the US1/LS1 pair of primers, specific for the original, published hSTAT3 sequence. 2) Genomic DNA amplified with the US3/LS3 pair of primers specific for the new variant hSTAT3 sequence. 3) Genomic DNA amplified with the US1/LS2 pair of primers, specific for the original, published hSTAT3 sequence. 4) Genomic DNA amplified with the US1/LS1 pair of primers. 5) Genomic DNA amplified with the US1/LS1 pair of primers and spiked with 0.3 fg of the US4/LS4-amplified artificial template. 6) Genomic DNA amplified with the US1/LS1 pair of primers and spiked with 3 fg of the US4/LS4-amplified artificial template. 7) Genomic DNA amplified with the US1/LS1 pair of primers and spiked with 30 fg of the US4/LS4-amplified artificial template. 8) Genomic DNA amplified with the US1/LS1 pair of primers and spiked with 300 fg of the US4/LS4-amplified artificial template.

### EXAMPLES

### MATERIALS AND METHODS

### Materials

HepG2 human hepatoma cell line was from ATCC (Rockville, MD, USA). Total human RNA from heart, liver, fetal liver, small intestine placenta and human genomic DNA were obtained from Clontech (Palo Alto, CA, USA). Other RNAs used in this study were prepared in our laboratory.

Pfu polymerase was from Stratagene (La Jolla, CA, USA); DNA Taq polymerase was from Advanced Biotechnology, Leatherhead, UK. DNA Sequencing Kit was from Perkin Elmer (Applied Biosystems Division, Foster City, CA, USA); SuperScript II reverse transcriptase (200 U/µl) and Trizol Reagent for RNA extraction were from Gibco (Grand Island, NY, USA).

### Oligonucleotide primers

In order to optimize the specificity of PCR amplification of template nucleotide sequences (even sequences differing by only a few nucleotides), all primers used in this study were designed in our laboratory with the help of the software OLIGO (National Biosciences, Plymouth, MN, USA).

All primers were also synthesized in our laboratory, with a 392 DNA/RNA Synthesizer from Perkin Elmer (Applied Biosystems Division, Foster City, CA, USA), to amplify various fragments in the SH2 domain of the hSTAT3 cDNA.

Two primers pairs, called US1/LS1 and US1/LS2, amplifying products of 285 and 111 bp respectively, were uniquely specific for the original published sequence of human STAT3 cDNA (Akira et al., 1994), and not for the hSTAT3 variant sequence we have found in this study. Single nucleotides at variance between the published and the variant STAT3 sequences were mainly positioned at the 3' end of each primer.

The nucleotide sequence of these primers are shown here below.
Primer US1: 5' TGA AGG GTA CAT CAT GGG TTT C 3' SEQ ID NO: 7
Primer LS1: 5' TCA- GGA TAG AGA TAG ACA AGT GGA GAC AA 3' SEQ ID NO: 8
Primer LS2: 5' CCT CCT TCT TTG CTG CTT- TCA CTG AAG 3' SEQ ID NO: 9

The US3/LS3 pair of primers was uniquely specific for the variant hSTAT3 sequence described in this study, with the following nucleotide sequence.
Primer US3: 5' CGA AGG GTA CAT CAT GGG CTT T 3' SEQ ID NO: 10
Primer LS3: 5' CCT CCT TCT- TTG CTG CTT TCA CTG AAT CTT 3' SEQ ID NO: 11

This US3/LS3 pair of primers did amplify a 111 bp fragment in the hSTAT3 variant sequence corresponding to the sequence amplified by the US2/LS2 primers in the original published hSTAT3 cDNA sequence.

A validation pair of primers, US4/LS4, to create an artificial hSTAT3 template of 285 bp corresponding to the product of primers US1/LS1, had the following sequence. Primer US4: 5' TGA AGG GTA CAT CAT GGG TTT CAT CAG TAA GGA 3' SEQ ID NO: 12 Primer LS4: 5' TCA- GGA TAG AGA TAG ACA AGT GGA GAC AAC AGG ATA T 3' SEQ ID NO: 13 **RNA and RT-PCR**

Total RNA from human HepG2 cells was prepared by the method of Birnboim (Birnboim, 1988).

For other tissues and cells RNA was extracted with the Trizol reagent available from Gibco, Grand Island, NY, USA, following manufacturer instructions. Oligo(dT) was used to prime reverse transcription of 5 µg of total RNA with 200 U of SuperScript II reverse transcriptase (RT) in 50 µl reaction mixture.

The RT reaction (Gram et al., 1992) was carried out at 37°C for 1h and 30 min.

Preparative PCR was then performed using the RT products as the DNA templates. PCR reactions contained 10 µl of cDNA, 50 pmoles of each primer (see below), 2,5 units of Stratagene Pfu polymerase, 0,2 mM of each of the four deoxynucleotide triphosphates, 10 µl of Pfu buffer, in a reaction volume of 100 µl, overlaid with 50 µl of mineral oil.

Amplification was performed for 30 cycles with a temperature profile of 45 seconds at 94°C (denaturation), 45 seconds at 60°C (annealing) and 5 minutes at 72°C (extention). PCR products were purified by centrifugation through Microcon 100 filters (Amicon) and then subjected to electrophoresis on 1,5 % agarose gels in Tris/borate/EDTA buffer.

Analytical PCRs were performed in capillary tubes, with the same concentration of reagents described above, but in ten-fold less volume, except for the Pfu polymerase which was substituted with 0.5 U of Taq polymerase. The temperature profile was 94°C for denaturation, 55°C for annealing and 72°C for extention.

### DNA sequencing

PCR products were sequenced with the same primers used for RT-PCR. DNA sequences were analyzed by the dideoxy method with the DNA sequencing kit (Perkin Elmer, Applied Biosystems Division, Foster City, CA, USA) on an ABI model 373A automated sequencer, following manufacturer instructions.

The nucleotide sequences of all cDNA fragments were determined from sequencing both DNA strands. Nucleotide and deduced amino acid sequences were compared with those in GenBank and the Swiss-Prot database.

### RESULTS AND DISCUSSION

### Isolation of a cDNA fragment from the SH2 domain of human STAT3

In order to isolate the SH2 domain of human STAT3, we have amplified by RT-PCR, using total RNA from HepG2 cells, a cDNA fragment of 424 base pairs corresponding to nucleotide positions between 1909 and 2332 of the published human placental STAT3 cDNA sequence (Akira et al., 1994).

This PCR fragment was then inserted in an expression vector, and the nucleotide sequence was determined. Results (Figure 1) showed that 13 nucleotide residues differed from the original human placental cDNA sequence.

The majority of the 13 modified residues were located in third codon position, resulting in no change of the corresponding amino acid sequence. Only one mutated nucleotide residue resulted in the substitution of a leucine at position 667 in the human STAT3 protein with a valine.

By considering only the SH2 fragment of 424 base pairs we have sequenced, the published human and mouse STAT3 nucleotide sequences differ by 31 residues (Akira et al., 1994; Raz et al., 1994; and Zhong et al., 1994).

The variant hSTAT3 sequence, that we have found, differs from the corresponding mouse sequence by 42 nucleotide residues in the same region. Thus, the new human STAT3 sequence fragment results in an increased evolutionary distance to the mouse sequence (Figure 1).

In addition, by considering the recently published genomic structure of the mouse STAT3 gene (Shi et al., 1996), the 13 nucleotide residues at variance between the two human STAT3 cDNA sequences, as determined in this study, fall in three different exons of the corresponding mouse genomic gene.

### Characterization of the new STAT3 sequence variant

In order to determine the nature of this sequence variant, we have designed three pairs of primers with 3' ends corresponding to nucleotide positions at variance between the two human cDNA sequences.

The first and the second pair of primers (US1/LS1 and US1/LS2)) were exclusively specific for the original published nucleotide sequence of the hSTAT3 cDNA, while the third pair of primers (US3/LS3) was exclusively specific for the new variant human STAT3 nucleotide sequence we have determined.

We have used the two primer pairs US1/LS1 and US3/LS3 (specific for the original and the new variant sequences respectively) to amplify RNAs from 11 different human tissues in 22 separate RT-PCR reactions. Each RNA source we have examined was derived from pools of 1 to 17 individuals, with a total of 31 individuals analyzed.

Since the original hSTAT3 cDNA sequence was derived from human placenta, this tissue was included among the 11 RNA sources tested. As shown in Figure 2, only the pair of primers specific for the new sequence variant were able to amplify all the eleven RNAs tested, resulting in the expected amplification product, while no significant band could be obtained in any RNA tested with the primers corresponding to the original published hSTAT3 sequence.

Since the US1/LS1 primers did not result in any significant amplification product, we wanted to verify whether this failure was due to a defect in the primers, either in their intrinsic ability to anneal to the appropriate template, or in their ability to prime the amplification reaction. In other words, we wanted to validate the US1/LS1 pair of primers.

Validation primers US4/LS4 were thus designed to match exactly primers US1/LS1, but each new primer with a 3' extention matching the hSTAT3 variant sequence determined in these experiments.

Amplification would then result in an artificial hybrid template composed of the hSTAT3 variant sequence fragment, with its 5' and 3' ends identical to primers US1 and LS1 respectively.

This artificial template should allow effective amplification with primers US1/LS1, even in the absence of the corresponding natural DNA template (i.e., the original, published hSTAT3 cDNA fragment of 285 bp).

This artificial template was obtained by PCR with primers US4/LS4, and spiked at different concentrations in human placental cDNA and in other human cDNAs. Primers US1/LS1 were then used to amplify these spiked cDNAs, and a PCR product of the expected size was readily obtained (Figure 3).

This result therefore excluded a failure of the US1/LS1 pair of primers in the amplification reaction.

We have then used the US1/LS1, US1/LS2 and US3/LS3 pairs of primers to amplify human genomic DNA. The expected amplification product was again obtained only with the primer pair specific for the new variant sequence we have determined (Figure 4).

In conclusion, we could not detect the hSTAT3 nucleotide sequence originally described by Akira et al. (Akira et al., 1994) in any of the human genomic or cDNA sources we have tested.

The original published nucleotide sequence and the new sequence variant are not therefore different genes or splice variants contemporaneously present in the same genome, since only one and the same one of these two sequences was always detected in each human nucleic acid source tested.

The two hSTAT3 sequence variants could be different alleles. In this case however, the new variant sequence is likely to be predominant, since it was represented in all nucleic acid samples tested, derived from a total of 31 individuals.

The original published hSTAT3 sequence was not represented at all in these individuals.

The new hSTAT3 variant nucleotide sequence described in this work corresponds to a small fragment of about 15% of the entire human STAT3 cDNA sequence.

### References

1. Akira, S., et al., (1994) Cell **77**, 63-71;
2. Azam, M., et al., (1995) EMBO Journal **14**, 1402-1411;
3. Birnboim, H.C. (1988) Nucleic Acids Research **16**, 1487-1497;
4. Gouilleux, F., et al., (1995) EMBO Journal **14**, 2005-2013;
5. Gram, H., et al., (1992) Proceedings of the National Academy of Sciences of the United States of America **89**, 3576-3580;
6. Harroch, S., et al., (1994) Journal of Biological Chemistry **269**, 26191-26195;
7. Hemmann, U., et al., (1996) Journal of Biological Chemistry **271**, 12999-13007;
8. Iwatsuki, K, et al., (1997) J. Biol. Chem **272**, 8149-8152;
9. Kapetein, A., et al., (1996) Journal of Biological Chemistry **271**, 5961-5964;
10.May, P., et al., (1996) FEBS Lett. **394**, 221-226;
11.Minami, M., et al., (1996) Proceedings of the National Academy of Sciences of the United States of America **93**, 3963-3966;
12.Quelle, F.W., et al., (1995) Molecular & Cellular Biology **15**, 3336-3343;
13.Raz, R., et al., (1994) Journal of Biological Chemistry **269**, 24391-24395;
14.Seidel, H.M., et al., (1995) Proceedings of the National Academy of Sciences of the United States of America **92**, 3041-3045;
15.Shi, W., et al., (1996) International Immunology **8**, 1205-1211;
16.Silva, C.M., et al., (1996) Molecular Endocrinology **10**, 508-518;
17.Stahl, N., et al., (1994) Science **263**, 92-95;
18.Stahl, N., et al., (1995) Science **267**, 1349-1353;
19.Tian, S.S., et al., (1994) Blood **84**, 1760-1764;
20.Wegenka, U.M., et al., (1993) Mol. Cell Biol. **13**, 276-288;
21.Wegenka, U.M., et al., (1994) Molecular & Cellular Biology **14**, 3186-3196;
22.Yamanaka, Y., et al., (1996) EMBO Journal **15**, 1557-1565;
23.Zhang, D., et al., (1996) Journal of Biological Chemistry **271**, 9503-9509;
24.Zhong, Z., et al., (1994) Science **264**, 95-98.

## Claims

1. A predominant allelic variant of human STAT3 protein.

2. A polypeptide comprising the amino acid sequence of SEQ ID NO:4, or a functionally equivalent salt, or a functionally equivalent derivative, or an active fraction, or a fusion protein.

3. A DNA molecule comprising a DNA sequence coding for the protein of claim 1.

4. A recombinant DNA molecule which hybridizes under stringent conditions with a probe having a nucleotide sequence selected between SEQ ID NO:10 and SEQ ID NO:11.

5. A DNA molecule comprising the nucleotide sequence of SEQ ID NO:3.

6. An expression vector which comprises the DNA molecule of any claims from 3 to 5.

7. A host cell comprising the expression vector of claim 6.

8. A recombinant process for preparing the protein of claim 1 or 2, comprising culturing in an appropriate culture medium the cells of claim 7.

9. A protein as defined in claim 1 or 2 for use as medicament.

10. Use of a protein according to claim 1 or 2 in the manufacture of a medicament for the treatment of autoimmune diseases or inflammatory illnesses.

11. A pharmaceutical composition comprising the protein defined in claim 1 or 2 together with one or more pharmaceutically acceptable carriers and/or excipients.
